# FASCICULE DE BREVET EUROPEEN

(11) **EP 0 983 750 B1**
(45) Date de publication et mention de la délivrance du brevet: **21.07.2004**
(21) Numéro de dépôt: 99420185.3
(22) Date de dépôt: 31.08.1999
(51) Int. Cl.: A61B 17/15, A61B 17/17

(54) **Matériel ancillaire de préparation rotulienne lors de la mise en place d'une prothèse**
Hilfsinstrument zur Vorbereitung des Einsetzens einer Kniescheiben-Prothese
Ancillary equipment for preparing the implanting of a prosthetic patella

(30) Priorité: 01.09.1998 FR 9811054
(43) Date de publication de la demande: 08.03.2000
(73) Titulaire: Jantet, Gilles, 69610 Meys (FR); Noyer, Daniel, 38300 Bourgoin Jallieu (FR)
(72) Inventeur: Noyer, Daniel, 38300 Bourgoin Jallieu (FR)
(74) Mandataire: Myon, Gérard Jean-Pierre

(56) Documents cités:
- EP-A- 0 737 444
- FR-A- 2 692 137

## Description

L'invention a trait à un matériel ancillaire de préparation rotulienne lors de la mise en place d'une prothèse de genou tricompartimentale ou fémoro-patélaire.

Lors de la mise en place d'une prothèse de genou tricompartimentale ou fémoro-patélaire, il convient de re-surfacer la face articulaire de la rotule destinée à venir en appui sur la trochlée d'un composant fémoral prothétique.

Il est notamment connu de mettre en place un insert ou bouton rotulien prothétique. Cet insert doit être mis en place d'une façon telle qu'il coopère avec la trochlée sans gêner les mouvements de flexion ou d'extension du genou.

Par la demande de brevet français 2 692 137, on connaît un dispositif de coupe rotulienne pour la pose d'une prothèse totale du genou dans lequel un téton de fixation est destiné à pénétrer dans un orifice latéral d'un composant fémoral d'essai, ce téton servant à supporter des moyens de coupe de la rotule parallèlement au plan de la trochlée. Ce dispositif nécessite l'utilisation d'un composant fémoral d'essai équipé d'un orifice latéral, ce qui n'est pas le cas de la plupart des composants existant sur le marché. L'utilisation des moyens de coupe de la rotule est donc limitée à une ou à quelques références de prothèses, voire à une marque de prothèse, ce qui peut ne pas être acceptable sur le plan économique pour un hôpital ou une clinique. En outre, dans le dispositif connu, la position des moyens de coupe par rapport au composant fémoral d'essai est fixe dans le plan sagittal. Il n'est pas possible d'ajuster la position de ces moyens de coupe, notamment de la broche de centrage de la coupe, en hauteur par rapport à la trochlée, voire par rapport au composant tibial de la prothèse. Il peut en résulter un mauvais positionnement de la rotule resurfacée par rapport à la prothèse totale ou partielle, pouvant conduire à des interférences entre les différents composants de la prothèse.

C'est à ces inconvénients qu'entend plus particulièrement remédier l'invention en proposant un matériel ancillaire compatible avec un grand nombre de types et de marques de prothèses, qu'il s'agisse de prothèses totales ou de prothèses fémoro-patélaires, ce matériel permettant un réglage antéropostérieur dans le plan sagittal de façon à garantir un positionnement optimal d'une broche de perçage et de guidage des moyens de coupe, en fonction des caractéristiques réelles du genou.

Dans cet esprit, l'invention concerne un matériel ancillaire de préparation rotulienne lors de la mise en place d'une prothèse de genou tricompartimentale ou fémoro-patélaire, ce matériel comprenant un composant fémoral d'essai présentant une trochlée prothétique, caractérisé en ce qu'il comprend une broche de guidage apte à être positionnée, par rapport audit composant fémoral d'essai, de façon pivotante autour d'un axe parallèle à l'axe d'articulation du genou, la broche étant coudée, de telle sorte que son pivotement induit un déplacement d'une extrémité libre de la broche dans un plan parallèle au plan sagittal du genou.

Grâce à l'invention, la broche peut guider des éléments de positionnement, tel qu'un viseur, pour la mise en place et l'utilisation de moyens de coupe de la rotule. Le fait que la broche pivote autour d'un axe parallèle à l'axe d'articulation du genou permet un réglage de la coupe de la rotule dans le plan sagittal.

Selon un premier aspect avantageux de l'invention, la broche a une extrémité apte à être insérée entre le composant fémoral d'essai et le fémur sur lequel il est monté. Cet aspect de l'invention permet de réaliser de façon particulièrement simple, fiable et économique le positionnement et le pivotement de la broche par rapport au composant fémoral d'essai dans trois plans de l'espace sagittal, frontal et horizontal.

Selon un autre aspect avantageux de l'invention, la broche est coudée à environ 90°. Cet aspect de l'invention permet qu'une branche de la broche se déplaçe dans un plan parallèle au plan sagittal du genou lors du pivotement de la broche.

Selon un autre aspect avantageux de l'invention, le matériel ancillaire comprend un viseur apte à être monté sur l'extrémité libre de la broche, ce viseur comprenant des moyens de réglage, perpendiculairement au plan sagittal du genou, de la position d'une broche de perçage et de guidage de moyens de coupe de la rotule. Grâce au viseur, la position de la broche de perçage et de guidage des moyens de coupe est ainsi réglable dans le plan sagittal, du fait du mouvement de pivotement de la broche de guidage, et perpendiculairement à ce plan, du fait de l'utilisation des moyens de réglage appartenant au viseur. Ainsi, le positionnement de la broche de perçage et de guidage de coupe peut-il être précisément défini en fonction des caractéristiques réelles de la rotule et des éléments prothétiques. Dans ce cas, on peut prévoir que les moyens de guidage comprennent au moins deux éléments formant guide disposés parallèlement les uns aux autres. Ces moyens de guidage sont particulièrement précis.

Selon un autre aspect avantageux de l'invention, le matériel comprend un viseur apte à être monté sur l'extrémité libre de la broche, ce viseur ou cette broche étant pourvu de moyens de blocage en rotation du viseur autour de l'extrémité libre de la broche. Cet aspect de l'invention garantit le bon positionnement du viseur, ainsi que de la broche de perçage et de guidage des moyens de coupe qu'il porte, par rapport aux plans sagittal, frontal et horizontal. On peut notamment prévoir que le viseur comprend un orifice de réception d'une branche portant l'extrémité libre de la broche et une fente de réception d'une partie de la broche non parallèle à cette branche portant l'extrémité libre. Cette construction permet un coincement du viseur sur la broche. Selon une autre variante, la broche peut comprendre deux extrémités libres portées par deux branches sensiblement parallèles alors que le viseur comprend deux orifices de réception de ces deux branches. Comme précédemment, le viseur est immobilisé en rotation.

Selon un autre aspect avantageux de l'invention, le matériel comprend une pince de fraisage de la rotule, cette pince étant apte à recevoir et à immobiliser la rotule alors qu'une broche traverse la rotule selon une direction antéro-postérieure. Cette pince permet un fraisage précis de la rotule après mise en place de la broche qui la traverse de part en part. L'épaisseur de fraisage est déterminée en fonction du réglage de cette pince.

L'invention sera mieux comprise et d'autres avantages de celle-ci apparaîtront plus clairement à la lumière de la description qui va suivre de deux modes de réalisation d'un matériel ancillaire de préparation rotulienne conforme à son principe, donnée uniquement à titre d'exemple et faite en référence aux dessins annexés dans lesquels :
- la figure 1 est une représentation schématique dans le plan sagittal de principe d'un genou équipé d'une prothèse tricompartimentale ;
- la figure 2 est une représentation schématique de principe en perspective d'une étape de pose de la prothèse de la figure 1 ;
- la figure 3 est une vue analogue à la figure 2 pour une seconde étape de pose ;
- la figure 4 est une vue en élévation avec arrachement partiel d'une broche et d'un viseur appartenant au matériel ancillaire conforme à l'invention ;
- la figure 5 est une vue en perspective d'une pince de fraisage appartenant au matériel ancillaire de l'invention et
- la figure 6 est une vue partielle, analogue à la figure 4, pour un matériel ancillaire conforme à un second mode de réalisation de l'invention.

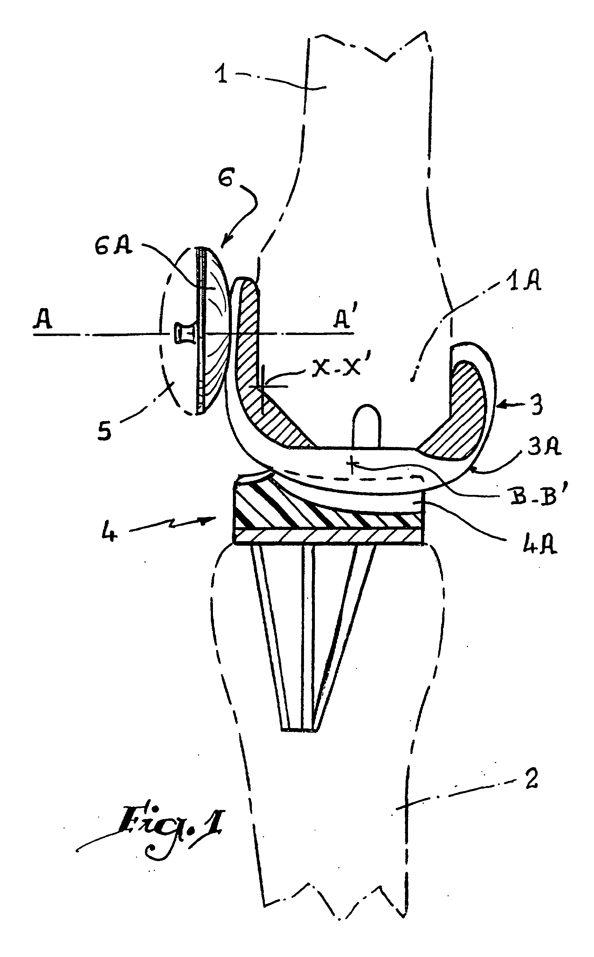

A la figure 1, un fémur 1 et un tibia 2 sont respectivement équipés de composants prothétiques 3 et 4 comprenant des surfaces d'articulation 3A et 4A disposées en regard. La rotule 5 est également équipée, sur sa face postérieure en regard du composant prothétique fémoral 3, d'un insert ou bouton 6 dont la surface extérieure 6A est prévue pour venir en appui sur la trochlée du composant prothétique fémoral 3.

L'axe AA' de la rotule 5 qui sert de référence pour l'usinage de cette rotule et la mise en place du bouton 6 doit être déterminé avec une très grande précision afin d'éviter que la surface articulaire rotulienne soit mal positionnée par rapport à la trochlée, voire vienne interférer avec le composant tibial 4. Cet axe AA' est globalement perpendiculaire à l'axe BB' d'articulation du genou.

La détermination de l'axe AA' a lieu de la façon suivante :

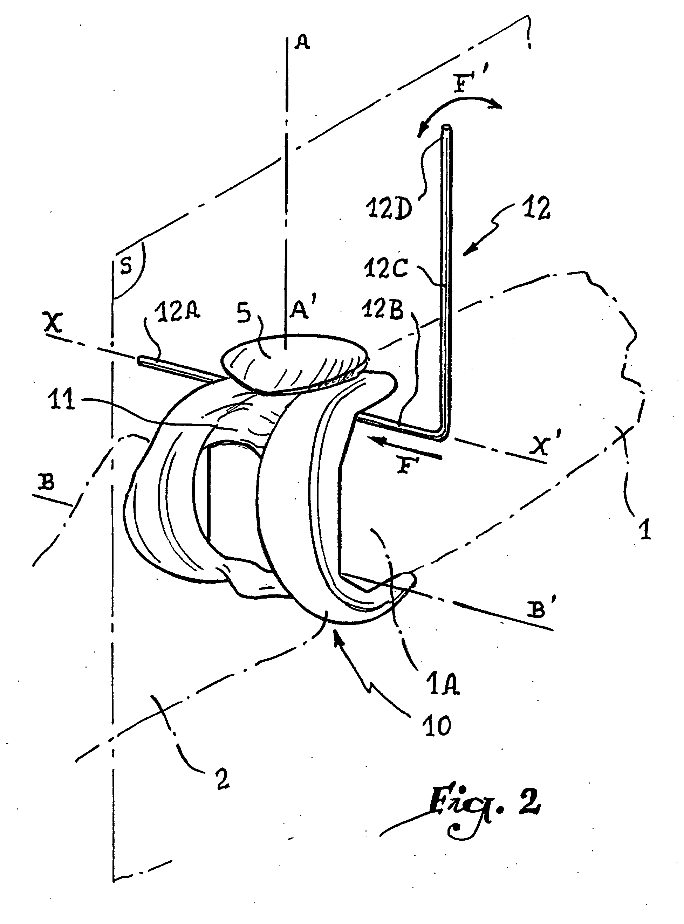

Après résection de l'extrémité inférieure 1A du fémur 1, un composant fémoral d'essai 10 est mis en place sur cette extrémité à la manière représentée à la figure 2, ce composant 10 comprenant une trochlée 11 sur laquelle peut être mise en appui la rotule 5. L'axe AA' de la rotule 5 doit être compris dans le plan sagittal S de l'articulation qui est le plan de la figure 1.

Une broche coudée 12 comprend une première extrémité 12A destinée à être insérée entre le composant 10 et l'extrémité 1A du fémur 1, comme représenté par la flèche F à la figure 2. On note 12B la branche de la broche 12 qui est glissée entre le composant 10 et le fémur 1. Cette branche est sensiblement rectiligne et à section globalement circulaire, de sorte qu'elle peut librement pivoter autour d'un axe XX' parallèle à l'axe BB' d'articulation du genou, comme représenté par la flèche F' à la figure 2. Ainsi, la seconde branche 12C de la broche 12, qui se termine par une extrémité libre 12D, se déplace dans un plan parallèle au plan sagittal S.

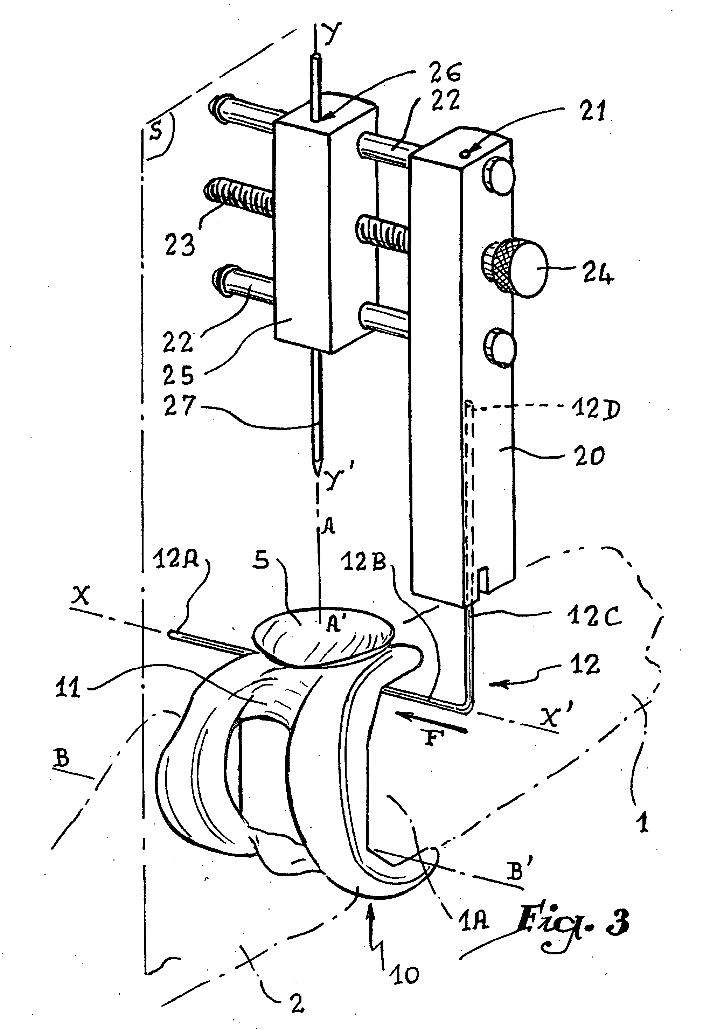

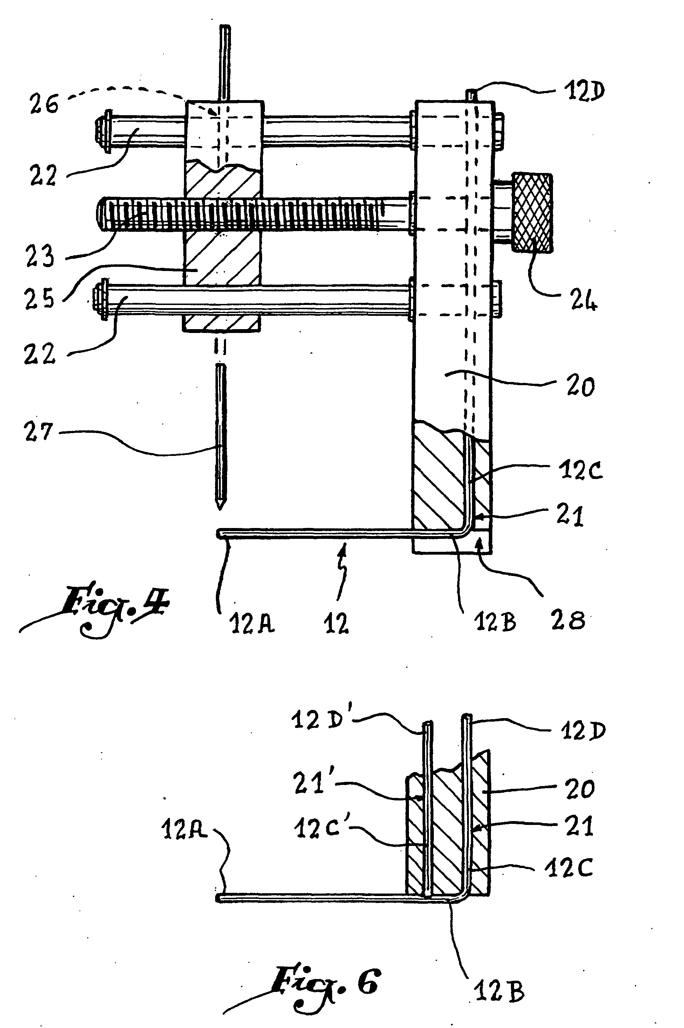

Comme il apparaît plus clairement aux figures 3 et 4, un viseur 20 peut être disposé sur la branche 12C de la broche 12. La branche 12C pénètre à l'intérieur d'un perçage ou orifice 21 du viseur 20. Le viseur 20 est équipé de deux barres sensiblement parallèles 22, et d'une tige filetée 23 commandée par une molette 24, la tige filetée 23 étant sensiblement parallèle aux barres 22 et servant également de guide.

Un bloc de visée 25 est monté sur les barres 22 et la tige filetée 23, la liaison entre la tige 23 et le bloc 25 étant du type vis-écrou. Ainsi, par action sur la molette 24, il est possible de déplacer le bloc de visée 25 perpendiculairement au plan sagittal S. Le bloc 25 est pourvu d'un perçage longitudinal 26 de réception d'une broche 27 prévue pour percer la rotule 5 et la traverser de part en part, c'est-à-dire de l'avant vers l'arrière. On note YY' l'axe de la tige 27 en position dans le bloc 25.

Un but essentiel de la présente invention est de faire coïncider le plus exactement possible l'axe YY' avec l'axe AA' de la rotule 5 en position "naturelle" sur la trochlée 11 du composant fémoral 10. Pour ce faire, il est possible d'ajuster la position du bloc 25 perpendiculairement au plan sagittal S en agissant sur la molette 24 pour disposer l'axe YY' dans le plan sagittal S. Ceci étant fait, on peut encore régler la position de l'axe YY' dans le plan sagittal en faisant pivoter la broche 12 autour de l'axe XX'.

Lorsque le chirurgien juge que les axes AA' et YY' sont confondus, il est possible d'impacter la broche 27 dans la rotule 5 sur une profondeur correspondant à la plus grosse partie de l'épaisseur de la rotule 5.

On peut alors retirer le viseur 20 de la broche 12 en tirant parallèlement à l'axe YY', de telle sorte que la broche 27 reste en place dans la rotule 5. Il est alors possible de coucher la rotule 5 sur le côté de façon à continuer l'impaction de la broche 27 jusqu'à ce que la broche traverse la rotule de part en part.

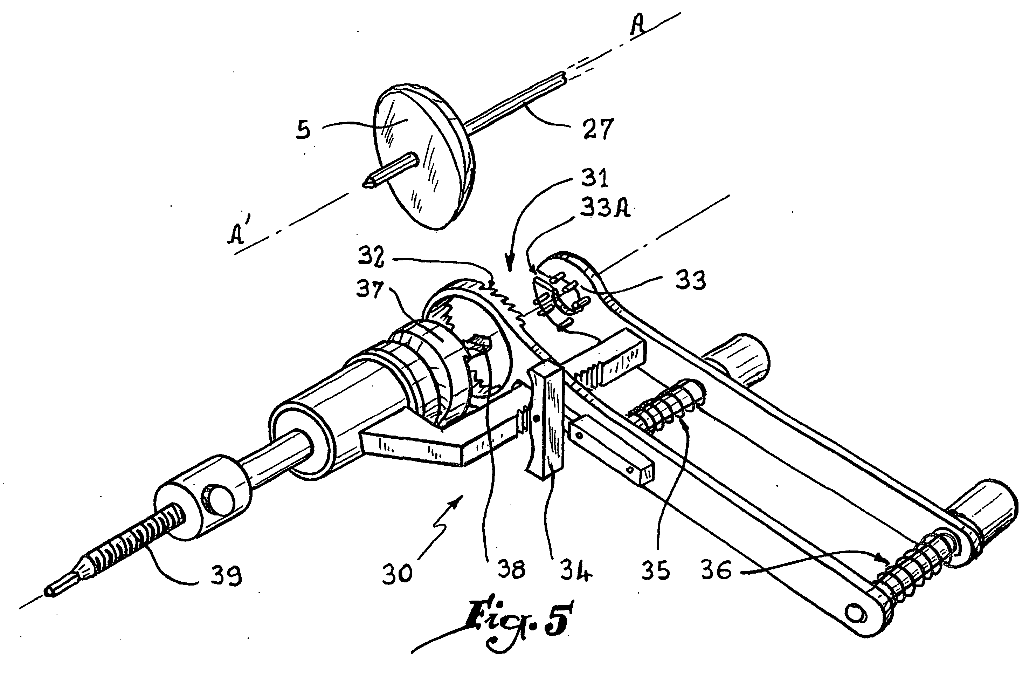

On utilise alors la pince 30 représentée à la figure 5 en introduisant la rotule dans une zone de réception 31 définie entre deux mâchoires 32 et 33 de la pince 30. On note que la mâchoire 33 est pourvue d'une fente 33A permettant le passage de la broche 27 lors de la mise en place de la rotule 5. La pince 30 est pourvue de moyens élastiques et/ou à cliquet 34, 35 et 36 de serrage de la rotule 5. Une fraise 37 est associée à la pince 30 et pourvue sur sa face avant d'un foret 38 qui est creux de façon à recevoir l'extrémité de la broche 27 qui dépasse de la face postérieure de la rotule 5. Ainsi, le foret 38 et la fraise 37 sont-ils correctement centrés par rapport à l'axe AA' de la rotule 5. Un moteur électrique ou un système manuel monté sur une tige d'entraînement 39 de la fraise 37 et du foret 38 permet d'actionner ces dispositifs.

A la place de la pince 30 adaptée au fraisage, il est possible d'utiliser un gabarit permettant une coupe par le côté de la rotule.

Aux figures 3 et 4, on note que le viseur 20 est pourvu d'une fente 28 dans laquelle peut être insérée la branche 12B de la broche 12 lorsque le viseur 20 est en appui sur cette broche. Ceci a pour effet d'immobiliser le viseur 20 en rotation autour de la branche 12C de la broche 12, ce qui garantit un positionnement adéquat de l'axe YY'.

Selon une variante de l'invention représentée à la figure 6, il est également possible de prévoir que la broche 12 comprend deux branches 12C et 12C' portant des extrémités libres 12D et 12D', et destinées à pénétrer respectivement dans deux orifices longitudinaux 21 et 21' du corps du viseur 20. Dans ce cas, il n'est pas nécessaire de prévoir de fente dans la partie inférieure du viseur dans la mesure où les deux branches 12C et 12C' et les deux orifices 21 et 21' concourent à l'immobilisation du viseur autour de chacune des branches 12C et 12C'.

La broche 12 a été représentée coudée à environ 90°, elle pourrait cependant être coudée de façon différente, la construction du viseur étant alors adaptée.

## Revendications

1. Matériel ancillaire de préparation rotulienne lors de la mise en place d'une prothèse de genou tricompartimentale ou fémoro-patélaire, ledit matériel comprenant un composant fémoral d'essai (10) présentant une trochlée prothétique (11), **caractérisé en ce qu'**il comprend une broche (12) de guidage apte à être positionnée, par rapport audit composant fémoral d'essai, de façon pivotante autour d'un axe (XX') parallèle à l'axe d'articulation (BB') du genou, ladite broche étant coudée, de telle sorte que son pivotement induit un déplacement d'une extrémité libre (12D) de ladite broche dans un plan parallèle au plan sagittal (S) du genou.

2. Matériel ancillaire selon la revendication 1, **caractérisé en ce que** ladite broche (12) a une extrémité (12A) apte à être insérée entre ledit composant fémoral d'essai (10) et le fémur (1) sur lequel il est monté.

3. Matériel ancillaire selon l'une des revendications 1 ou 2, **caractérisé en ce que** ladite broche (12) est coudée à environ 90°.

4. Matériel ancillaire selon l'une des revendications précédentes, **caractérisé en ce qu'**il comprend un viseur (20) apte à être monté sur l'extrémité libre de ladite broche (12), ledit viseur comprenant des moyens de réglage (22-24), perpendiculairement au plan sagittal (S) du genou, de la position d'une broche (27) de perçage de la rotule (5) et de guidage de moyens de coupe (37, 38).

5. Matériel ancillaire selon la revendication 4, **caractérisé en ce que** lesdits moyens (22-24) de guidage comprennent au moins deux éléments (22-23) formant guide disposés parallèlement les uns aux autres.

6. Matériel ancillaire selon l'une des revendications précédentes, **caractérisé en ce qu'**il comprend un viseur (20) apte à être monté sur l'extrémité libre (12D) de ladite broche (12), ledit viseur et/ou ladite broche étant pourvu de moyens (28 ; 12C, 12C', 21, 21') de blocage en rotation dudit viseur autour de ladite extrémité libre de ladite broche.

7. Matériel ancillaire selon la revendication 6, **caractérisé en ce que** ledit viseur (20) comprend un orifice (21) de réception d'une branche (12C) portant ladite extrémité libre (12D) de ladite broche (12) et une fente (28) de réception d'une partie (12B) de ladite broche non parallèle à ladite banche portant ladite extrémité libre.

8. Matériel ancillaire selon la revendication 6, **caractérisé en ce que** ladite broche (12) comprend deux extrémités libres (12D, 12D') portées par deux branches (12C, 12C') sensiblement parallèles et **en ce que** ledit viseur comprend deux orifices (21, 21') de réception desdites deux branches.

9. Matériel ancillaire selon l'une des revendications précédentes, **caractérisé en ce qu'**il comprend une pince (30) de fraisage de la rotule (5), ladite pince étant apte à recevoir et à immobiliser la rotule alors qu'une broche (27) traverse la rotule selon une direction antéro-postérieure (AA').

## Patentansprüche

1. Hilfsinstrument zur Kniescheibenvorbereitung beim Einsetzen einer dreiteiligen oder Femur-Patella Knieprothese, wobei das Instrument ein Schenkelprobenteil (10) umfasst, das eine prothetische Trochlea (11) aufweist, **dadurch gekennzeichnet, dass** es einen Führungsstift (12) umfasst, der geeignet ist, in Bezug auf das Schenkelprobenteil in schwenkender Weise um eine Achse (XX') parallel zur Drehachse (BB') des Knies positioniert zu werden, wobei der Stift abgewinkelt ist, derart, dass sein Schwenken eine Verschiebung eines freien Endes (12D) des Stiftes in einer Ebene parallel zur Sagittalebene (S) des Knies induziert.

2. Hilfsinstrument nach Anspruch 1, **dadurch gekennzeichnet, dass** der Stift (12) ein Ende (12A) aufweist, das geeignet ist, zwischen das Schenkelprobenteil (10) und den Schenkelknochen (1), an dem es montiert ist, eingefügt zu werden.

3. Hilfsinstrument nach einem der Ansprüche 1 oder 2, **dadurch gekennzeichnet, dass** der Stift (12) um ungefähr 90° abgewinkelt ist.

4. Hilfsinstrument nach einem der vorhergehenden Ansprüche, **dadurch gekennzeichnet, dass** es eine Visiereinrichtung (20) umfasst, die geeignet ist, auf das freie Ende des Stiftes (12) montiert zu werden, wobei die Visiereinrichtung Mittel (22-24) zur Einstellung eines Stiftes (27) zum Durchbohren der Kniescheibe und zum Führen von Schneidmittel (37, 38) senkrecht zur Sagittalebene (S) des Knies umfasst.

5. Hilfsinstrument nach Anspruch 4, **dadurch gekennzeichnet, dass** die Mittel (22-24) zum Führen mindestens zwei die Führung bildende Elemente (22-23) umfassen, die parallel zueinander angeordnet sind.

6. Hilfsinstrument nach einem der vorhergehenden Ansprüche, **dadurch gekennzeichnet, dass** es eine Visiereinrichtung (20) umfasst, die geeignet ist, auf das freie Ende des Stiftes (12) montiert zu werden, wobei die Visiereinrichtung und/oder der Stift mit Mitteln (28, 12C, 12C', 21, 21') zur Verdrehsicherung der Visiereinrichtung um das freie Ende des Stiftes versehen sind.

7. Hilfsinstrument nach Anspruch 6, **dadurch gekennzeichnet, dass** die Visiereinrichtung (20) eine Öffnung (21) zur Aufnahme eines das freie Ende (12D) des Stiftes (12) tragenden Armes und einen Spalt (28) zur Aufnahme eines Teils (12B) des Stiftes, das nicht parallel zu dem das freie Ende tragenden Armes angeordnet ist, umfasst.

8. Hilfsinstrument nach Anspruch 6, **dadurch gekennzeichnet, dass** der Stift (12) zwei frei Enden (12D, 12D') umfasst, die von zwei im wesentlichen parallel angeordneten Armen (12C, 12C') getragen werden und dass die Visiereinrichtung zwei Öffnungen (21, 21') zur Aufnahme der zwei Arme umfasst.

9. Hilfsinstrument nach einem der vorhergehenden Ansprüche, **dadurch gekennzeichnet, dass** es eine Zange (30) zum Fräsen der Kniescheibe (5) umfasst, wobei die Zange in der Lage ist, die Kniescheibe aufzunehmen und festzuhalten, wobei ein Stift (27) die Kniescheibe entsprechend einer Richtung Vorderseite-Rückseite (A, A') durchquert.

## Claims

1. Ancillary equipment for preparing the patella when fitting a tricompartmental or patellofemoral knee prosthesis, the said equipment comprising a test femoral component (10) having a prosthetic trochlea (11), **characterised in that** it contains a guide pin (12) suitable for positioning, with respect to the said test femoral component, in a manner such that it pivots about an axis (XX') parallel to the axis of articulation (BB') of the knee, the said pin being bent such that pivoting thereof gives rise to movement of a free end (12D) of the said pin in a plane parallel to the sagittal plane (S) of the knee.

2. Ancillary equipment according to Claim 1, **characterised in that** the said pin (12) has an end (12A) suitable for insertion between the said test femoral component (10) and the femur (1) on which it is fitted.

3. Ancillary equipment according to one of Claims 1 or 2, **characterised in that** the said pin (12) is bent at approximately 90°.

4. Ancillary equipment according to one of the preceding claims, **characterised in that** it has a viewfinder (20) suitable for mounting on the free end of the said pin (12), the said viewfinder having means ( 22 - 24) for adjustment, perpendicularly to the sagittal plane (S) of the knee, of the position of a pin (27) for perforating the patella (5) and for guiding cutting means (37, 38).

5. Ancillary equipment according to Claim 4, **characterised in that** the said guide means (22 - 24) comprise at least two elements (22 - 23) forming a guide arranged parallel to one another.

6. Ancillary equipment according to one of the preceding claims, **characterised in that** it has a viewfinder (20) suitable for mounting on the free end (12D) of the said pin (12), the said viewfinder and/or the said pin being provided with means (28; 12C, 12C', 21, 21') for blocking rotation of the said viewfinder about the said free end of the said pin.

7. Ancillary equipment according to Claim 6, **characterised in that** the said viewfinder (20) has an orifice (21) for accommodating an arm (12C) having the said free end (12D) of the said pin (12) and a slot (28) for accommodating a part (12B) of the said pin that is not parallel to the said formwork (sic) having the said free end.

8. Ancillary equipment according to Claim 6, **characterised in that** the said pin (12) has two free ends (12D, 12D') on two essentially parallel arms (12C, 12C') and **in that** the said viewfinder has two orifices (21, 21') for accommodating the said two arms.

9. Ancillary equipment according to one of the preceding claims, **characterised in that** it has a clamp (30) for milling the patella (5), the said clamp being suitable for accommodating and immobilising the patella while a pin (27) passes through the patella in an antero-posterior direction (AA').
